Europäisches Patentamt

(19) European Patent Office

Office européen des brevets

(11) EP 0 494 972 B1

(12) **EUROPEAN PATENT SPECIFICATION**

(45) Date of publication and mention
of the grant of the patent:
**27.11.1996 Bulletin 1996/48**

(21) Application number: **90915661.4**

(22) Date of filing: **13.09.1990**

(51) Int. Cl.$^6$: **A61K 9/46**

(86) International application number:
**PCT/US90/05206**

(87) International publication number:
**WO 91/04757 (18.04.1991 Gazette 1991/09)**

(54) **EFFERVESCENT DOSAGE FORM AND METHOD OF ADMINISTERING SAME**

BRAUSEDOSISFORM SOWIE VERFAHREN ZU DEREN VERABREICHUNG

PRESENTATION SOUS FORME EFFERVESCENTE D'UN MEDICAMENT A USAGE PEDIATRIQUE

(84) Designated Contracting States:
**AT BE CH DE DK ES FR GB IT LI LU NL SE**

(30) Priority: **02.10.1989 US 416152**
**11.04.1990 US 507642**

(43) Date of publication of application:
**22.07.1992 Bulletin 1992/30**

(60) Divisional application: **96201072.4**

(73) Proprietor: **CIMA LABS, INC.**
**Minneapolis, MN 55428 (US)**

(72) Inventors:
• **WEHLING, Fred**
**New Hope, MN 55427 (US)**
• **SCHUEHLE, Steve**
**Minneapolis, MN 55413 (US)**
• **MADAMALA, Navayanarao**
**Plymouth, MN 55442 (US)**

(74) Representative: **Mossmark, Anders et al**
**Albihn West AB,**
**Box 142**
**401 22 Göteborg (SE)**

(56) References cited:
**EP-A- 0 219 337          EP-A- 0 313 328**
**EP-A- 0 396 335          FR-A- 2 190 408**
**GB-A- 0 013 160          US-A- 4 687 662**
**US-A- 4 725 427**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.8/3.4

**Description**

<u>Technical Field</u>

The present invention relates to the field of orally ingested solid dosage forms.

<u>Background Art</u>

One challenge in pharmacy is that many people are unwilling and/or unable to swallow tablets, capsules or other traditional solid dosage forms. In particular, children generally do not like to take medicine, vitamins, minerals or dietary supplements. Most children dislike medicine because of its flavor. This problem becomes particularly acute when the medicine, vitamin, mineral or dietary supplement must be taken on a daily basis.

In an attempt to make medicine, vitamins, minerals, dietary supplements, and the like, more palatable to children, a number of techniques have been employed. Many pediatric medicines are formulated with large amounts of sweeteners and flavorants to mask the taste of the active ingredients. For example, common children's multivitamin pills include sweeteners and flavorants together with vitamins and minerals. U.S. Patent No. 2,887,437 relates to a palatable vitamin tablet containing an amino acid. The tablet is designed to be swallowed whole, chewed without objectionable taste, dissolved in the mouth, or dissolved in liquids. It contains a plurality of vitamins, a nutritionally essential amino acid, a flavoring agent, and a hydrophilic starch as a disintegration agent. Flavored disintegrable pills have, however, been generally ineffective in overcoming children's reluctance to taking medicines and particularly vitamins which generally require daily administration. While these pills are less objectionable than other dosage forms, the flavor is often overpowered by the taste of the medicine.

One approach to the administration of medicines, vitamins, minerals, and the like to persons in general is the use of effervescent tablets.

Effervescence can be defined as the evolution of bubbles of gas in a liquid. As set forth in Chapter 6 of <u>Pharmaceutical Dosage Forms: Tablets Volume I 2nd Edition. A. Lieberman</u>, ed. 1989, Marcel Dekker, Inc. (the entirety of which is hereby incorporated by reference), effervescent mixtures have been known and used medicinally for many years. As discussed in this text, and as commonly employed, an effervescent tablet is dissolved in water to provide a carbonated or sparkling liquid drink. In such a drink the effervescence helps to mask the taste of medicaments. However, the use of effervescent tablets to prepare a beverage including medicaments, is not convenient. It requires preparatory steps before administration of the drug and also requires the presence of a suitable mixing container.

In a departure from the traditional use of effervescence, U.S. Patent No. 4,639,368 describes a chewing gum containing a medicament capable of absorption through the buccal cavity and a composition capable of generating carbon dioxide as a taste masking agent. The gum may optionally include a further taste bud desensitizing compound. Unfortunately there are substantial disadvantages inherent in such a gum based delivery system. Many medicants are not suited for buccal absorption. Gums are difficult to prepare. Because of braces or other dental work, many persons are not permitted to chew gum. Furthermore, the medicament must be released into solution in the saliva. Thus the full taste of the medicament is perceived, subject only to the taste masking effect. If the flavor and/or the effervescent taste masker are insufficient and/or fade prior to the full release of medicament, the patient will be left with a gum having an objectionable taste. Gums also leave residues which must be disposed of.

Effervescent tablets have also been used in the dental area. Westlake, U.S. Patent No. 1,262,888, Howell, U.S. Patent No. 3,962,417 and Aberg U.S. Patent No. 4,753,792 disclose effervescent dentifrice tablets adapted to foam in the mouth of a patient so as to provide a tooth cleansing action.

Chavkin U.S. Patent No.4,613,497 discloses a water foamable pharmaceutical composition incorporating an effervescent agent, a polysaccheride gum, and a gelling salt together with a pharmaceutically active ingredient. The composition is not intended to immediately disintegrate but rather to form a stable foam in the patient's stomach or other body cavity so that the active ingredient is gradually released from the foam.

In order to administer a rapidly acting pharmaceutical ingredient to a patient, it is known from, for example, Ashmead, U. S. Patent No. 4,725,427 and Schobel, U. S. Patent No. 4,687,662 to provide effervescent water-soluble compositions which are dissolved in water to provide a drink. To make the drink more palatable, i.e. to disguise the unpleasant taste of the medicament, flavorings are added.

A chewable tablet is known from EP-A-0 396 335, a document falling under Art. 54(3) EPC, which comprises a medicament dispersed in a chewable base, such as mannitol, together with an effervescent couple, such as citric acid - sodium bicarbonate. The combination of effervescence and chewability with optional flavourings is said to improve the taste characteristics of the medicament in oral administration. A disintegrant such as microcrystalline celulose may be added to give the patient the option of dispersing the tablet in water.

EP-A-0 313 328 is concerned with ways of presenting pharmacologically active substances in a sustained-release form, for example over a period of 12 to 24 hours. According to said document, this is achieved by the provision of granules in a tablet, with the granules comprising a core of one or more pharmacologically active substances and a coating covering substantially the whole surface of the core and comprising a water insoluble but water swellable acrylic polymer and a water soluble hydroxylated cellulose derivative.

Despite these and other efforts towards developments of suitable dosage forms, there have been unmet needs heretofore for improved dosage forms for the administration of systemically distributable pharmaceutical ingredients such as drugs, vitamins and the like. There also remains a need for a convenient and effective dosage form for intended ingredients which may be consumed by all children, including those who can't chew a gum or swallow a pill and will be readily accepted thereby.

Summary of the Invention

The present invention addresses these needs.

One aspect of the present invention provides a solid dosage form adapted for direct oral administration to a human patient comprising: a mixture of at least one water or saliva activated effervescent disintegration agent and a plurality of microcapsules, said microcapsules including at least one systemically distributable pharmaceutical ingredient and an encapsulant substantially surrounding the pharmaceutical ingredient, said microcapsules providing rapid release of said at least one pharmaceutical ingredient, said mixture being in the form of a tablet of a size and shape adapted for direct oral administration to a human patient, said tablet being substantially completely disintegrable upon exposure to water or saliva to release said microcapsules, with said at least one effervescent disintegration agent being present in an amount which is effective to aid in rapid disintegration of said tablet without chewing, and to provide a distinct sensation of effervescence upon disintegration of the tablet in the mouth of the patient.

The effervescent sensation is not only pleasant to the patient but also tends to stimulate saliva production, thereby providing additional water to aid in further effervescent action. Thus, once the tablet is placed in the patient's mouth, it will disintegrate rapidly and substantially completely without any voluntary action by the patient. The systemically distributable ingredient is thus carried into solution or into suspension in the patient's own saliva, which the patient ordinarily swallows. Even if the patient does not chew the tablet, disintegration will proceed rapidly. Therefore, dosage forms according to the aspect of the present invention are particularly useful in administration of medications to individuals who cannot or will not chew, such as debilitated patients, patients who have difficulty swallowing solids, and the elderly.

According to a particularly preferred aspect of the invention, the systemically distributable ingredient is a pharmaceutical ingredient including at least one psychotropic drug such as a sedative, antidepressant, neuroleptic, or hypnotic. The present invention is especially valuable with psychotropic drugs in that a patient receiving such drugs, particularly a patient in a mental institution, often attempts to hold a conventional tablet or capsule concealed within his mouth rather than swallow it. The patient may then surreptitiously remove the tablet or capsule when medical personnel are not present. The preferred dosage forms according to this aspect of the present invention are substantially resistant to such concealment, inasmuch as they will disintegrate rapidly even if they are concealed within the mouth.

According to the present invention, the systemically distributable pharmaceutical ingredient is provided in microencapsulated form. Thus, the mixture includes microcapsules each incorporating a systemically distributable pharmaceutical ingredient and an encapsulant surrounding the pharmaceutical ingredient. Upon disintegration of such a tablet, the individual microcapsules are released and dispersed in the patient's mouth in admixture with the patient's saliva whereupon the microcapsules are conducted to the digestive tract for systemic distribution.

The combination of the effervescent disintegration agent and the microcapsules provides a uniquely effective dosage form for systemically distributable pharmaceutical ingredients which have unpleasant flavors or which should not be released within the mouth for other reasons. Because the tablet will disintegrate without chewing, the problem of microcapsule rupture during chewing of the tablet is substantially eliminated. Stated another way, the effervescent action allows administration of the tablet without chewing, so as to maintain the efficacy of the microencapsulant.

Best Mode For Carrying Out the Invention

An oral dosage form according to one embodiment of the present invention is a tablet of a size and shape adapted for direct oral administration. The mass of each such tablet generally should be less than about 3.0 g and more preferably less than about 1.5 g. The tablet may include surface markings, cuttings, grooves, letters and or numerals for the purpose of decoration and/or identification. The tablet is, of course, in solid form. Preferably, the tablet is a hard compressed tablet. It includes one or more systemically distributable ingredients, together with an effervescent disintegrating agent. The size of the tablet is also dependent upon the amount of material used, however, it is preferable to provide tablets which have a largest dimension of about 1.75 cm (11/16 inches).

When used for children, the tablet may have the shape of letters, numbers, animals, birds, cartoon characters, fish, dinosaurs, and the like. Further, the tablet may include surface markings, cuttings, grooves, letters and or numerals for the purpose of decoration and/or identification. The tablet is, of course, in solid form. Preferably, the tablet is a hard compressed tablet. It includes one or more intended ingredients, together with an effervescent disintegrating agent.

The term "systemically distributable ingredient" as used in this disclosure should be understood to mean an ingredient(s), the ingestion of which is the reason for consuming a tablet in which the ingredient is included. These ingredients are conducted from the mouth to the digestive system for absorption through the stomach or intestines and systemic distribution through the bloodstream. The term "pharmaceutical ingredient" is not intended to be limited to pharmaceutical ingredients which are systemically active or which systemically distribute over time. For the purposes of the present invention, a systemically distributable ingredient may include pharmaceuticals or minerals, vitamins and dietary supplements. Mixtures of any of the foregoing are also contemplated by the term systemically distributable pharmaceutical ingredient.

By the term pharmaceutical(s) applicants mean a drug. Pharmaceutical(s) may include, without limitation, antacids, analgesics, anti-inflammatories, antibiotics, laxatives, anorexics, antiasthmatics, antidiuretics, antiflatuents, antimigraine agents, antispasmodics, sedatives, antihyperactives, tranquilizers, antihistamines, decongestants, beta blockers, and combinations thereof.

As used in this disclosure, the term vitamin refers to trace organic substances that are required in the diet. For the purposes of the present invention, the term vitamin(s) include, without limitation, thiamin, riboflavin, nicotinic acid, pantothenic acid, pyrdoxine, biotin, folic acid, vitamin B12, lipoic acid, ascorbic acid, vitamin A, vitamin D, vitamin E and vitamin K. Also included within the term vitamin are the coenzymes thereof. Coenzymes are specific chemical forms of vitamins. Coenzymes include thiamine pyrophosphates (TPP), flavin mononucleotide (FMM), flavin adenine dinucleotive (FAD), Nicotinamide adenine dinucleotide (NAD), Nicotinamide adenine dinucleotide phosphate (NADP) Coenzyme A (CoA) pyridoxal phosphate, biocytin, tetrahydrofolic acid, coenzyme B12, lipoyllysine, *11-cis*-retinal, and 1,25 dihydroxycholecalciferol. The term vitamin(s) also includes choline, carnitine, and alpha, beta, and gamma carotenes.

As used in this disclosure, the term "mineral" refers to inorganic substances, metals, and the like required in the human diet. Thus, the term "mineral" as used herein includes, without limitation, calcium, iron, zinc, selenium, copper, iodine, magnesium, phosphorus, chromium and the like, and mixtures thereof.

The term "dietary supplement" as used herein means a substance which has an appreciable nutritional effect when administered in small amounts. Dietary supplements include, without limitation, such ingredients as bee pollen, bran, wheat germ, kelp, cod liver oil, ginseng, and fish oils, amino-acids, proteins and mixtures thereof. As will be appreciated, dietary supplements may incorporate vitamins and minerals.

The amount of systemically distributable ingredient incorporated in each tablet may be selected according to known principles of pharmacy. An effective amount of systemically distributable ingredient is specifically contemplated. By the term effective amount, it is understood that, with respect to for example pharmaceuticals, a pharmaceutically effective amount is contemplated. A pharmaceutically effective amount is the amount or quantity of a drug or pharmaceutically active substance which is sufficient to elicit the required or desired therapeutic response, or in other words, the amount which is sufficient to elicit an appreciable biological response when administered to a patient. As used with reference to a vitamin or mineral, the term "effective amount" means an amount at least 10% of the United States Recommended Daily Allowance ("RDA") of that particular ingredient for a patient. For example, if an intended ingredient is vitamin C, then an effective amount of vitamin C would include an amount of vitamin C sufficient to provide 10% or more of the RDA. Typically, where the tablet includes a mineral or vitamin, it will incorporate higher amounts, preferably 100% or more of the applicable RDA.

The term effervescent disintegration agent(s) includes compounds which evolve gas. The preferred effervescent agents evolve gas by means of chemical reactions which take place upon exposure of the effervescent disintegration agent to water and/or to saliva in the mouth.

The bubble or gas generating reaction is most often the result of the reaction of a soluble acid source and an alkali metal carbonate or carbonate source. The reaction of these two general classes of compounds produces carbon dioxide gas upon contact with water included in saliva.

Such water activated materials must be kept in a generally anhydrous state with little or no absorbed moisture or in a stable hydrated form since exposure to water will prematurely disintegrate the tablet. The acid sources or acid may be any which are safe for human consumption and may generally include food acids, acid anhydrides and acid salts. Food acids include citric acid, tartaric acid, malic acid, fumaric acid, adipic acid, and succinic acids etc. Because these acids are directly ingested, their overall solubility in water is less important than it would be if the effervescent tablet formulations of the present invention were intended to be dissolved in a glass of water. Acid anhydrides and acid of the above described acids may also be used. Acid salts may include sodium, dihydrogen phosphate, disodium dihydrogen pyrophosphate, acid citrate salts and sodium acid sulfite.

Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate.

The effervescent disintegration agent(s) of the present invention is not always based upon a reaction which forms carbon dioxide. Reactants which evolve oxygen or other gasses which are pediatrically safe are also considered within the scope. Where the effervescent agent includes two mutually reactive components, such as an acid source and a carbonate source, it is preferred that both components react completely. Therefore, an equivalent ratio of components which provides for equal equivalents is preferred. For example, if the acid used is diprotic, then either twice the amount of a mono-reactive carbonate base, or an equal amount of a di-reactive base should be used for complete neutralization to be realized. However, in other embodiments of the present invention, the amount of either acid or carbonate source may exceed the amount of the other component. This may be useful to enhance taste and/or performance of a tablet containing an overage of either component. In this case, it is acceptable that the additional amount of either component may remain unreacted.

In general, the amount of effervescent disintegration agent of the present invention useful for the formation of tablets according to the present invention should range from 5 to 50% by weight of the final composition, and preferably between 15 and 30% by weight thereof. In a more preferred embodiment, the amount of effervescent disintegration agent according to the present invention ranges from between 20 and 25% by weight of the total composition.

More specifically, tablets according to the present invention should contain an amount of effervescent disintegration agent effective to aid in the rapid and complete disintegration of the tablet when orally administered. By "rapid", it is understood that the tablets of the present invention should disintegrate in the mouth of a patient in less than 10 minutes, and desirably between 30 seconds and 7 minutes. In a particularly preferred embodiment according to the present invention, the tablet should dissolve in the mouth in between 30 seconds and 5 minutes. Disintegration time in the mouth can be measured by observing the disintegration time of the tablet in water at about 37°C. The tablet is immersed in the water without forcible agitation. The disintegration time is the time from immersion for substantially complete dispersion of the tablet as determined by visual observation. As further discussed below, tablets according to the invention, include microcapsules. These may be insoluble or more slowly soluble than the tablet binder. As used in this disclosure the term "complete disintegration" of the tablet does not require dissolution or disintegration of such microcapsules or other discrete inclusions. Disintegration times referred to in this disclosure should be understood as determined by this method unless otherwise specified.

Also, the amount of effervescent disintegration agent present in the tablet should be effective to provide an effervescent sensation in the mouth of the patient who consumes the tablet. Thus, the patient should be able to perceive a distinct sensation of "fizzing" or bubbling as the tablet disintegrates in the mouth. To provide this sensation, the amount of effervescent agent in each tablet desirably is arranged to provide 20 to 60 cm$^3$ of gas. The "fizzing" sensation substantially enhances the organoleptic effects of the tablet. Thus, the amount of effervescent disintegration agent useful in accordance with the present invention is also an amount effective to provide a positive organoleptic sensation to a patient. A "positive" organoleptic sensation is one which is pleasant or enjoyable and which can be perceived readily by a normal human being.

It should also be noted that the hardness of a tablet may also play a role in disintegration time. Specifically, increasing the hardness of a tablet may increase the disintegration time just as decreasing hardness may decrease disintegration time.

The dosage form according to this aspect of the present invention may further include one or more additional adjuvants which can be chosen from those known in the art including flavors, dilutents, colors, binders, filler, compaction vehicles, and non effervescent disintegrants.

Examples of binders which can be used include acacia, tragacanth, gelatin, starch, cellulose materials such as methyl cellulose and sodium carboxy methyl cellulose, alginic acids and salts thereof, magnesium aluminum silicate, polyethylene glycol, guar gum, polysaccharide acids, bentonites, sugars, invert sugars and the like. Binders may be used in an amount of up to 60 weight percent and preferably 10 to 40 weight percent of the total composition.

Non-effervescent disintegrants include starches as corn starch, potato starch and modified starches thereof, sweeteners, clays, such as bentonite, micro-crystalline cellulose, alginates, gums such as agar, guar, locust bean, karaya, pecitin and tragacanth. Disintegrants may comprise up to 20 weight percent and preferably between 2 and 10 percent of the total weight of the composition.

Coloring agents may include titanium dioxide, and dyes suitable for food such as those known as F.D.& C. dyes and natural coloring agents such as grape skin extract, beet red powder, beta-carotene, annato, carmine, turmeric, paprika, etc.. The amount of coloring used may range from 0.1 to 3.5 weight percent of the total composition.

Flavors, incorporated in the composition may be chosen from synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavors are vanilla, citrus oil, including lemon, orange, grape, lime and grapefruit, and fruit essences, including apple pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors may be present in an amount ranging from 0.5 to 3.0% by weight based upon the weight of the composition. Particularly preferred flavors are the orange, grape and cherry flavors.

Tablets according to this aspect of the present invention can be manufactured by well-known tableting procedures. In common tableting processes, material which is to be tableted is deposited into a cavity, and one or more punch members are then advanced into the cavity and brought into intimate contact with the material to be pressed, whereupon compressive force is applied. The material is thus forced into conformity with the shape of the punches and the cavity. Hundreds, and even thousands, of tablets per minute can be produced in this fashion. Various tableting methods, well known to those skilled in the art, are comprehensively discussed in Lieberman, Pharmaceutical Dosage Forms: Tablets Volume 1, Second Edition, Revised and Expanded Copyright 1989 by Marcel Dekker, Inc.

Materials to be delivered are often pretreated either alone or in combination with other fillers to form granules that readily lend themselves to tableting. This process is known as granulation. As commonly defined, "granulation" is any process of size enlargement whereby small particles are gathered together into larger, permanent aggregates to yield a free-flowing composition having a consistency suitable for tableting. Such granulated compositions may have consistency similar to that of dry sand. Granulation may be accomplished by agitation in mixing equipment or by compaction, extrusion or globulation. In granulation, active or intended ingredients are generally admixed with a compression vehicle incorporating the effervescent disintegration agent and other adjuvants referred to above. The compression vehicle or filler should have good compressibility, good flowability and stability under normal ambient conditions as well as being low in cost and satisfactory in both texture and appearance.

As noted in Chapter 6 of Pharmaceutical Dosage Forms, supra, lubricants normally are used in manufacture of effervescent tablets. Without the use of an effective lubricant, tableting by use of today's high speed equipment would not be possible. Effervescent granulations are inherently difficult to lubricate due to both the nature of the raw materials and the requirement that the tablets disintegrate rapidly.

Lubricant, as used herein, means a material which can reduce the friction arising at the interface of the tablet and the die wall during compression and ejection thereof. Lubricants may also serve to prevent sticking to the punch and, to a lesser extent, the die wall as well. The term "antiadherents" is sometimes used to refer specifically to substances which function during ejection. As used in the present disclosure, however, the term "lubricant" is used generically and includes "antiadherents". Tablet sticking during formation and/or ejection may pose serious production problems such as reduced efficiency, irregularly formed tablets, and non-uniform distribution of intended agents or ingredients to be delivered thereby. These problems are particularly severe with high speed tableting approaches and methods.

Lubricants may be intrinsic or extrinsic. A lubricant which is directly applied to the tableting tool surface in the form of a film, as by spraying onto the die cavity and/or punch surfaces, is known as an extrinsic lubricant. Although extrinsic lubricants can provide effective lubrication, their use requires complex application equipment and methods which add cost and reduce productivity.

Intrinsic lubricants are incorporated in the material to be tableted. Magnesium, calcium and zinc salts of stearic acid have long been regarded as the most efficient intrinsic lubricants in common use. Concentrations of one percent or less are usually effective.

Other traditional intrinsic lubricants include hydrogenated and partially hydrogenated vegetable oils, animal fats, polyethyleneglycol, polyoxyethylene monostearate, talc, light mineral oils, sodium benzoate, sodium lauryl sulphate, magnesium oxide and the like. See European Patent Application No. 0,275,834. See also Leal, et al., U.S. Patent No. 3,042,531.

Lubricants, according to the present invention, may be used in an amount of up to 1.5 weight percent and preferably between 0.5 and 1.0 weight percent of the total composition.

Intrinsic lubricants pose certain serious difficulties when used in conventional tablets. Many lubricants materially retard the disintegration of non-effervescent tablets. However, the effervescent disintegration agents used in the dosage form of the present invention overcome any such retardation. In dissolution of conventional effervescent tablets, the lubricant may cause "scumming" and/or agglomeration. Stearates, for example leave an objectionable "scum" when an effervescent tablet is placed in a glass of water. This "scum" reduces the aesthetic appeal of the solution made from an effervescent dosage form. However, because the tablets of the present invention dissolve in the mouth, the solution is never seen by the user. Therefore, the propensity of a lubricant to "scum" is unimportant. Thus, lubricants which can cause dissolution or scumming problems in other dosage forms can be used in dosage forms according to the present invention without material adverse effect.

Microencapsulation of the systemically distributable pharmaceutical ingredient may be used to encapsulate both solids and liquids. Microencapsulation has been used in chewable tablets by coating drug particles or droplets with an edible polymeric material. The microcapsules help to mask the taste of the drug and minimize physical and chemical incompatibilities between ingredients. However, upon compression of the tablet, and during mastication, the microcapsule may become ruptured. As a result, the patient is then exposed to the otherwise objectionable tasting medicine. See Lieberman. Pharmaceutical Dosage Forms: Tablets Volume 1, at pages 372-376.

The present invention provides a solution to this problem in two important ways. First, to the extent that the tablet is held in the mouth and not chewed, destruction of the microcapsules is avoided. Furthermore, to the extent that microcapsules do break, the effervescent action of the invention provides additional taste masking.

Furthermore, tablets, according to the present invention contain microcapsules which have a specific time-release profile. By the use of the present invention, the microencapsulated systemically distributable pharmaceutical ingredient is delivered to the digestive system as a suspension or slurry of microcapsules. Thus, in calculating the time release profile of the encapsulant, no additional estimation of the time necessary for the tablet's dissolution will be required. This allows for both a higher degree of accuracy in drug delivery and a high degree of convenience for the patient.

The material selected as an encapsulant substantially prevents dissolution of individual microcapsules within a patient's mouth. Any type of rapid release microcapsule may be incorporated into the formulation in accordance with the present invention. These may include microcapsules using a semi permeable mechanism for rapid release provided by reaction of specific chemicals contained within the digestive track. In a preferred embodiment in accordance with the present invention, dissolvable encapsulant is used, such as, for example, ethylcellulose which dissolves in intestinal fluid. When such polymeric formulations are used they are typically applied to solid ingredient particles by a conventional spray coating method in an amount which may be as much as 50% by weight. Typically, however, 3-15% by weight, based on the weight of the powder, and most typically 5%-10% by weight are used. When liquid ingredients are encapsulated, conventional interfacial condensation reactions may be used. Other conventional methods are well known in the art.

The foregoing will be better understood with reference to the following examples which detail a particularly preferred procedure for the manufacture of tablets according to the present invention. All references made to these examples are for the purposes of illustration. They are not to be considered limiting as to the scope and nature of the present invention.

EXAMPLE 1

| ACETAMINOPHEN 325 MG EFFERVESCENT TABLET | |
|---|---|
| INGREDIENTS | AMOUNT PER 1000 TABLETS |
| * acetaminophen | 350.5 gm |
| sorbitol | 400.0 gm |
| compressible sugar binder | 400.0 gm |
| citric acid | 125.0 gm |
| sodium bicarbonate | 100.0 gm |
| cherry flavor powder | 6.0 gm |
| aspartame | 40.0 gm |
| monopotassium phosphate | 25.0 gm |
| lubricant | 25.0 gm |
| | 1,471.5 gm |

*Acetaminophen powder spray coated with 7% ethylcellulose by Eurand America Corporation.

Directly mix all ingredients in a suitable blender. Discharge and compress on a tablet press to form a tablet which weights 1,471.5 mg.

EXAMPLE 2

| AMITRIPTYLINE HCL 50 MG EFFERVESCENT TABLET ||
| INGREDIENTS | AMOUNT PER 1000 TABLETS |
| --- | --- |
| *amitriptyline HCl | 53.7 gm |
| sorbitol | 400.0 gm |
| compressible sugar binder | 400.0 gm |
| citric acid | 125.0 gm |
| sodium bicarbonate | 100.0 gm |
| cherry flavor powder | 6.0 gm |
| aspartame | 40.0 gm |
| monopotassium phosphate | 25.0 gm |
| lubricant | 25.0 gm |
| | 1,174.7 gm |

*Amitriptyline HCl powder spray coated with 7% ethylcellulose.

Directly mix all ingredients in a suitable blender. Discharge and compress on a tablet press to form a tablet which weights 1,174.7 mg.

EXAMPLE 3

| FLURAZEPAM HCL 15 MG EFFERVESCENT TABLET ||
| INGREDIENTS | AMOUNT PER 1000 TABLETS |
| --- | --- |
| *flurazepam HCl | 16.1 gm |
| sorbitol | 400.0 gm |
| compressible sugar binder | 400.0 gm |
| citric acid | 125.0 gm |
| sodium bicarbonate | 100.0 gm |
| cherry flavor powder | 6.0 gm |
| aspartame | 40.0 gm |
| monopotassium phosphate | 25.0 gm |
| lubricant | 25.0 gm |
| | 1,137.1 gm |

*Flurazepam HCl powder spray coated with 7% ethylcellulose.

Directly mix all ingredients in a suitable blender. Discharge and compress on a tablet press to form a tablet which weighs 1,137.1 mg.

EXAMPLE 4

| CHLORDIAZEPOXIDE HCL 25 MG EFFERVESCENT TABLET | |
|---|---|
| INGREDIENTS | AMOUNT PER 1 000 TABLETS |
| *chlordiazepoxide HCl | 26.9 gm |
| sorbitol | 400.0 gm |
| compressible sugar binder | 400.0 gm |
| citric acid | 125.0 gm |
| sodium bicarbonate | 100.0 gm |
| cherry flavor powder | 6.0 gm |
| aspartame | 40.0 gm |
| monopotassium phosphate | 25.0 gm |
| lubricant | 25.0 gm |
| | 1,147.9 gm |

*chlordiazepoxide HCl powder spray coated with 7% ethylcellulose.

Directly mix all ingredients in a suitable blender. Discharge and compress on a tablet press to form a tablet which weighs 1,147.9 mg.

The principles, preferred embodiments and modes of operation of the present invention have been described in the foregoing specification. The invention which is intended to be protected herein, however, is not to be construed as limited to the particular embodiments disclosed, since these are to be regarded as illustrative rather than restrictive.

**Claims**

1. A solid dosage form adapted for direct oral administration to a human patient comprising: a mixture of at least one water or saliva activated effervescent disintegration agent and a plurality of microcapsules, said microcapsules including at least one systemically distributable pharmaceutical ingredient and an encapsulant substantially surrounding the pharmaceutical ingredient, said microcapsules providing rapid release of said at least one pharmaceutical ingredient, said mixture being in the form of a tablet of a size and shape adapted for direct oral administration to a human patient, said tablet being substantially completely disintegrable upon exposure to water or saliva to release said microcapsules, with said at least one effervescent disintegration agent being present in an amount which is effective to aid in rapid disintegration of said tablet without chewing, and to provide a distinct sensation of effervescence upon disintegration of the tablet in the mouth of the patient.

2. A dosage form as claimed in claim 1, characterized in that said at least one effervescent disintegration agent is present in said tablet in an amount sufficient to provide at least 20 cm$^3$ of a gas upon activation of said effervescent agent with water.

3. A dosage form as claimed in claim 1 or 2, characterized in that said at least one effervescent disintegration agent is present in said tablet in an amount sufficient to provide between 20 and 60 cm$^3$ of said gas upon activation of said at least one effervescent agent by water.

4. A dosage form as claimed in claim 1, 2 or 3, characterized in that said at least one systemically distributable ingredient comprises a pharmaceutical ingredient including at least one psychotropic drug.

5. A dosage form as claimed in claim 4, characterized in that at least one psychotropic drug includes at least one drug selected from the group consisting of sedatives, antidepressants, neuroleptics, and hypnotics.

**Patentansprüche**

1. Dosierung in fester Form, die zur direkten oralen Verabreichung an einen menschlichen Patienten ausgelegt ist, umfassend:
eine Mischung aus wenigstens einem durch Wasser oder Speichel aktivierten brausenden Zerfallsmittels und einer Vielzahl von Mikrokapseln, wobei die Mikrokapseln zumindest einen systemisch verteilbaren pharmazeutischen Inhaltsstoff und ein den pharmazeutischen Inhaltsstoff im wesentlichen umschließendes Einbettungsmittel enthalten, die Mikrokapseln eine schnelle Freisetzung des zumindest einen pharmazeutischen Inhaltsstoffes ermöglichen, die Mischung in Form einer Tablette in einer Größe und Form vorliegt, die zur direkten oralen Verabreichung an einen menschlichen Patienten ausgelegt ist, die Tablette nach Einwirken von Wasser oder Speichel im wesentlichen vollständig zerfallen kann, um die Mikrokapseln freizusetzen, wobei das zumindest eine brausende Zerfallsmittel in einer solchen Menge vorliegt, die wirksam ist, um einen schnellen Zerfall der Tablette ohne Kauen zu ermöglichen, und um einen deutlichen Brauseeindruck beim Zerfall der Tablette im Mund des Patienten zu erzeugen.

2. Dosierform nach Anspruch 1,
dadurch gekennzeichnet, daß
das zumindest eine brausende Zerfallsmittel in der Tablette in einer solchen Menge vorliegt, die ausreichend ist, um zumindest 20 cm$^3$ eines Gases bei Aktivierung des Brausemittels mit Wasser bereitzustellen.

3. Dosierform nach Anspruch 1 oder 2,
dadurch gekennzeichnet, daß
das zumindest eine brausende Zerfallsmittel in der Tablette in einer solchen Menge vorliegt, die ausreichend ist, um zwischen 20 und 60 cm$^3$ des Gases bei Aktivierung des zumindest einen Brausemittels durch Wasser bereitzustellen.

4. Dosierform nach Anspruch 1, 2 oder 3,
dadurch gekennzeichnet, daß
der zumindest eine systemisch verteilbare Inhaltsstoff einen pharmazeutischen Inhaltsstoff aufweist, der zumindest einen psychotropen Wirkstoff enthält.

5. Dosierform nach Anspruch 4,
dadurch gekennzeichnet, daß
der zumindest eine psychotrope Wirkstoff zumindest einen Wirkstoff enthält, ausgewählt aus der Gruppe, bestehend aus Sedativa, Antidepressiva, Neuroleptika und Hypnotika.

**Revendications**

1. Présentation pharmaceutique solide conçue pour l'administration orale directe à un malade humain, comprenant :
un mélange d'au moins un agent de désintégration effervescent activé par l'eau ou par la salive et d'une pluralité de microcapsules, lesdites microcapsules comprenant au moins un composant pharmaceutique susceptible de distribution systémique et un encapsulant entourant sensiblement le composant pharmaceutique, lesdites microcapsules assurant une libération rapide dudit composant pharmaceutique au nombre minimal de 1, ledit mélange se présentant sous la forme d'un comprimé de grosseur et forme conçues pour l'administration orale directe à un malade humain, ledit comprimé étant susceptible de désintégration sensiblement complète lors d'exposition à de l'eau ou à la salive pour libérer lesdites microcapsules, ledit agent de désintégration effervescent au nombre minimal de 1 étant présent en une quantité qui est efficace pour contribuer à une désintégration rapide dudit comprimé en l'absence de mastication et pour fournir une sensation distincte d'effervescence lors de la désintégration du comprimé dans la bouche du malade.

2. Présentation pharmaceutique selon la revendication 1, caractérisée en ce que ledit agent de désintégration effervescent au nombre minimal de 1 est présent dans ledit comprimé en une quantité suffisante pour fournir au moins 20 cm$^3$ d'un gaz lors de l'activation dudit agent effervescent par de l'eau.

3. Présentation pharmaceutique selon la revendication 1 ou 2, caractérisée en ce que ledit agent de désintégration effervescent au nombre minimal de 1 est présent dans ledit comprimé en une quantité suffisante pour fournir entre 20 et 60 cm$^3$ dudit gaz lors de l'activation dudit agent effervescent au nombre minimal de 1 par de l'eau.

4. Présentation pharmaceutique selon la revendication 1, 2 ou 3, caractérisée en ce que ledit composant susceptible de distribution systémique, au nombre minimal de 1, comprend un composant pharmaceutique comprenant au moins un médicament psychotrope.

5. Présentation pharmaceutique selon la revendication 4, caractérisée en ce que le médicament psychotrope au nombre minimal de 1 comprend au moins un médicament sélectionné dans le groupe constitué par les sédatifs, les anti-dépresseurs, les neuroleptiques et les hypnotiques.